# EUROPÄISCHE PATENTSCHRIFT

(11) **EP 1 157 673 B1**
(45) Veröffentlichungstag und Bekanntmachung des Hinweises auf die Patenterteilung: **07.03.2007**
(21) Anmeldenummer: 01106959.8
(22) Anmeldetag: 20.03.2001
(51) Int. Cl.: A61F 2/84

(54) **Stent, Positionierelement und Einführkatheter**
Stent, position's element and delivery catheter
Endoprothèse, élément de positionnement et cathéter de mise en place

(30) Priorität: 26.05.2000 DE 10026307
(43) Veröffentlichungstag der Anmeldung: 28.11.2001
(73) Patentinhaber: Variomed AG, 9496 Balzers (LI)
(72) Erfinder: Fenesi, Peter, 76275 Ettlingen (DE)
(74) Vertreter: Manitz, Finsterwald & Partner GbR

(56) Entgegenhaltungen:
- EP-A- 0 968 688
- EP-A- 1 000 590
- EP-A- 1 036 550
- WO-A-94/22379
- US-A- 5 413 586
- US-A- 5 695 499
- US-A- 5 861 027

## Beschreibung

Die vorliegende Erfindung betrifft einen Stent zur transluminalen Implantation in Hohlorgane, insbesondere in Blutgefäße, Urogenitaltrakt, Gastrointestinaltrakt, iatrogen Shunt, Pulmonalindikationen oder Gallenwege, mit einem im wesentlichen röhrenförmigen Grundkörper, der von einem komprimierten Zustand mit einem minimalem Querschnittsdurchmesser in einen expandierten Zustand mit einem vergrößerten Querschnittsdurchmesser überführbar ist. Weiterhin ist die Erfindung auf ein Positionierelement für einen Stent sowie auf einen Einführkatheter für einen Stent gerichtet.

Stents der eingangs genannten Art werden üblicherweise in ihrem komprimierten Zustand auf einen Mandrin eines Einführkatheters aufgesetzt und anschließend mit einer auf den Mandrin aufgezogenen Außenhülle auf dem Mandrin fixiert. Der Stent wird mit dem Einführkatheter an die gewünschte Stelle innerhalb des Hohlorgans positioniert, woraufhin zum Aussetzen des Stents die Außenhülle zurückgezogen wird. Handelt es sich um einen selbstexpandierenden Stent aus einer Formgedächtnislegierung, so expandiert der Teil des Stents, von dem die Außenhülle zurückgezogen wird, automatisch, so daß nach vollständigem Zurückziehen der Außenhülle der Stent aufgrund der Körpertemperatur seinen expandierten Zustand aufnimmt. Handelt es sich bei dem Stent nicht um einen selbstexpandierenden Stent, so wird der Übergang von dem komprimierten Zustand in den expandierten Zustand beispielsweise durch Ballondilatation, d.h. durch Aufblasen eines zwischen dem Mandrin und dem Stent vorhandenen Ballons erreicht.

Insbesondere bei selbstexpandierenden Stents kann das Problem auftreten, daß der Stent aufgrund der bei der Selbstexpandierung auftretenden Kräfte eigenständig aus der Außenhülle herausspringt, bevor die Außenhülle kontrolliert vollständig zurückgezogen ist. In diesem Fall kann die Außenhülle nicht mehr über den freigegebenen expandierten Stent aufgeschoben werden kann. Eine Repositionierung des Stents ist somit üblicherweise nicht mehr möglich. Weiterhin kann dieses Springen des Stents zu Fehlplazierungen des Stents insbesondere in distaler Richtung führen.

Ein Stent und ein Positionierelement, welche dieses Problem lösen, sind z.B. aus EP-A-1 000 590 bekannt.

Es ist eine Aufgabe der vorliegenden Erfindung gemäß Anspruch 1, einen Stent, ein Positionierelement für einen Stent sowie einen Einführkatheter für einen Stent anzugeben, mit denen neben einer exakten Positionierung auch eine Repositionierung möglich ist, wobei zusätzlich durch ein Springen des Stents verursachte Fehlplazierungen zuverlässig vermieden werden sollen und wobei das Positionierelement einen möglichst geringen Durchmesser haben soll.

Der den Stent betreffende Teil der Aufgabe wird ausgehend von einem Stent der eingangs genannten Art dadurch gelöst, daß zumindest an einem Ende des Grundkörpers in Umfangsrichtung verteilt mehrere Halteelemente ausgebildet sind, die jeweils einen sich vom Ende des Grundkörpers aus erstreckenden Verbindungsabschnitt und einen sich daran anschließenden Halteabschnitt aufweisen, wobei jeder Halteabschnitt in Umfangsrichtung eine größere Breite aufweist als sein Verbindungsabschnitt und im komprimierten Zustand des Stents zwischen in Umfangsrichtung benachbart angeordneten Halteabschnitten jeweils ein Freiraum ausgebildet ist.

Ein erfindungsgemäß ausgebildetes Positionierelement für einen Stent mit Halteelementen umfaßt einen ringförmigen Körper, in dem in Umfangsrichtung verteilt mehrere, Aufnahmeabschnitte für die Halteelemente bildende Ausnehmungen ausgebildet sind, die sich jeweils bis zu einem stirnseitigen Ende des ringförmigen Körpers erstrecken und dort in die Umgebung münden, wobei jeweils der zur Mitte des ringförmigen Körpers hin gelegene Bereich der Ausnehmungen in Umfangsrichtung eine größere Breite aufweist als der zum stirnseitigen Ende hin gelegene Bereich. Ein erfindungsgemäßer Einführkatheter mit einem innenliegenden Mandrin und einer auf dem Mandrin verschiebbaren Außenhülle ist durch ein unverschiebbar auf dem Mandrin angeordnetes, erfindungsgemäßes Positionierelement gekennzeichnet.

Ein erfindungsgemäßer Stent besitzt somit an zumindest einem seiner Enden Halteelemente, die mit einem entsprechenden Positionierelement so zusammenwirken, daß die Halteelemente in Ausnehmungen an dem Positionierelement eingehängt werden können. Dabei sind die Halteelemente und das Positionierelement so ausgebildet, daß eine Entkopplung des komprimierten Stents und des Positionierelements in Längsrichtung des Stents nicht möglich ist. Die Halteelemente des Stents können lediglich in radialer Richtung aus den Ausnehmungen des Positionierelements austreten, so daß eine feste Kopplung zwischen dem Stent und dem Positionierelement solange gewährleistet ist, solange sich zumindest der Endbereich des Stents mit den Halteelementen im komprimierten Zustand befindet.

Demgemäß wird der Stent im komprimierten Zustand auf den Mandrin eines Einführkatheters so aufgebracht, daß die Halteelemente in den Ausnehmungen des Positionierelements zu liegen kommen. Anschließend wird die Außenhülle über den Mandrin und den Stent geschoben, so daß der komprimierte Zustand des Stents durch die Außenhülle gehalten wird.

Nachdem der Stent mit dem Einführkatheter an die gewünschte Position innerhalb des Hohlorgans verbracht wurde, wird die Außenhülle zur Freigabe des distalen Endes des Stents zurückgezogen, wie es aus dem Stand der Technik bekannt ist. Solange die Außenhülle noch das Positionierelement mit den darin eingelegten Halteelementen des Stents überdeckt, kann der Stent nicht vollständig aus der Außenhülle austreten, so daß ein Springen des Stents zuverlässig verhindert wird.

Weiterhin ist es aufgrund des fest mit dem Positionierelement verkoppelten Stentendes möglich, die Außenhülle bei einer Fehlpositionierung vorsichtig vorzuschieben oder den Mandrin zurückzuziehen, so daß der bereits großteils expandierte Stent wieder in die Außenhülle zurückziehbar ist und neu positioniert werden kann.

Erst nachdem die Außenhülle vollständig zurückgezogen ist, d.h. daß das distale Ende der Außenhülle auch die in den Ausnehmungen des Positionierelements angeordneten Halteelemente frei gibt, können diese radial nach außen aus den Ausnehmungen des Positionierelements austreten, so daß der Stent vollständig freigegeben und positioniert ist. Da kurz vor diesem Zeitpunkt bereits der größte Teil des Stents vollständig expandiert ist und somit an der Innenwand des Hohlorgans anliegt, ist eine Verschiebung des Stents und damit eine Fehlpositionierung aufgrund eines Springen des Stents ausgeschlossen.

In dieser Anmeldung wird der Begriff "distal" mit der Bedeutung "vom Körper der behandelnden Person weg gelegen" verwendet, während der Begriff "proximal" entsprechend mit der Bedeutung "zum Körper der behandelnden Person hin gelegen" verwendet wird.

Bevorzugt besitzen die Halteabschnitte eine abgerundete Kontur. Dadurch ist gewährleistet, daß durch den Stent keine Verletzungen des Hohlorgans, beispielsweise der Wand einer Ader, erfolgen kann.

Weiterhin ragen die Außenseiten der Halteelemente vorteilhaft nicht über die radiale Außenkontur des Grundkörpers hinaus. Auch dies trägt zur Vermeidung von Verletzungen der Organwände bei.

Nach einer vorteilhaften Ausführungsform der Erfindung ragen die Innenseiten der Halteelemente nicht über die radiale Innenkontur des Grundkörpers in dessen Inneres hinein. Auf diese Weise wird ein ungehinderter Fluß der jeweiliger Körperflüssigkeit, beispielsweise von Blut innerhalb einer Ader gewährleistet.

Bevorzugt erstrecken sich die Halteelemente in Längsrichtung des Stents, wodurch ein besonders einfaches Angreifen der Halteelemente ermöglicht wird.

Der Außenkontur der Halteelemente kann beispielsweise pilzförmig ausgebildet sein, da eine solche Form in günstiger Weise die erforderliche abgerundete Kontur mit verbreiterten Halteabschnitten verbindet.

Nach einer weiteren vorteilhaften Ausführungsform der Erfindung ist die Dicke der Halteelemente in radialer Richtung im wesentlichen konstant. Die Halteabschnitte können unterschiedlich groß oder alle im wesentlichen gleich groß ausgebildet sein.

Vorteilhaft besitzen die Halteelemente, insbesondere die Halteabschnitte, eine größere Röntgendichte als der Grundkörper des Stents. Auf diese Weise kann die jeweilige Position des Stents beim Einsetzen am Röntgenschirm beobachtet werden.

Die Ausnehmungen des Positionierelements sind gemäß der Erfindung als Durchbrechungen in der Außenseite des ringförmigen Körpers ausgebildet. Wesentlich ist lediglich, daß die Ausnehmungen so ausgebildet sind, daß die eingesetzten Halteelemente des Stents in Längsrichtung nicht aus den Ausnehmungen herausgezogen werden können, sondern lediglich in radialer Richtung aus den Ausnehmungen entfernt werden können.

Bevorzugt münden alle Ausnehmungen an demselben stirnseitigen Ende des ringförmigen Körpers in die Umgebung. Grundsätzlich ist es jedoch auch möglich, daß manche der Ausnehmungen oder alle Ausnehmungen an beiden stirnseitigen Enden des ringförmigen Körpers in die Umgebung münden. Auf diese Weise kann der Stent mit seinen Halteelementen von beiden Seiten des ringförmigen Körpers her an diesen angekoppelt werden.

Bevorzugt sind die Ausnehmungen im wesentlichen komplementär zu der Form der Halteelemente des Stents ausgebildet. Beispielsweise kann die Kontur der Ausnehmungen pilzförmig ausgebildet sein.

Durch die komplementäre Form wird eine formschlüssige Kopplung zwischen dem Positionierelement und dem Stent erreicht, so daß der Stent fest, d.h. ohne Spiel, mit dem Positionierelement verbunden ist.

Nach einer weiteren vorteilhaften Ausführungsform der Erfindung besteht der ringförmige Körper aus einem im wesentlichen röntgendichten Material, beispielsweise aus Platin. Da der ringförmige Körper beim Einsetzen des Stents aufgrund seiner Haltefunktion unmittelbar am proximalen Ende des Stents angeordnet ist, kann somit die Position des Stents durch die am Röntgenschirm beobachtbare Position des ringförmigen Körpers sehr gut verfolgt werden. Da der ringförmige Körper eine wesentlich größere Materialdichte besitzt als der Stent, ist somit die Röntgenbeobachtbarkeit beim Einsetzen des Stents verbessert.

Nach einer weiteren vorteilhaften Ausführungsform der Erfindung sind zumindest ein Teil der Ausnehmungen, insbesondere alle Ausnehmungen voneinander getrennt ausgebildet. Grundsätzlich ist es jedoch auch möglich, daß ein Teil der Ausnehmungen oder alle Ausnehmungen untereinander verbunden sind. Beispielsweise kann der zur Mitte hin gelegene Bereich der Ausnehmungen als in Umfangsrichtung verlaufende Ringnut ausgebildet sein, von der aus jeweils Kanäle zu einer oder beiden Stirnseiten des Rings hin verlaufen. In diesem Fall würden die breiter ausgebildeten Halteabschnitte der Halteelemente in der Ringnut positioniert, während die Verbindungsabschnitte jeweils in den Kanälen angeordnet sind.

Weitere vorteilhafte Ausführungsformen der Erfindung sind in den Unteransprüchen angegeben.

Die Erfindung wird nachfolgend anhand eines Ausführungsbeispiels unter Bezugnahme auf die Zeichnungen näher beschrieben; in diesen zeigen:
- Fig. 1: eine Abwicklung eines erfindungsgemäß ausgebildeten Stents,
- Fig. 2: eine Abwicklung eines erfindungsgemäß ausgebildeten Positionierelements,
- Fig. 3: einen Schnitt durch das Positionierelement nach Fig. 2,
- Fig. 4: eine Detailansicht eines mit einem Positionierelement gemäß den Fig. 2 und 3 gekoppelten Stents nach Fig. 1,
- Fig. 5: einen erfindungsgemäß ausgebildeten Einführkatheter und
- Fig. 6: eine Detailansicht des Einführkatheters aus Fig. 5.

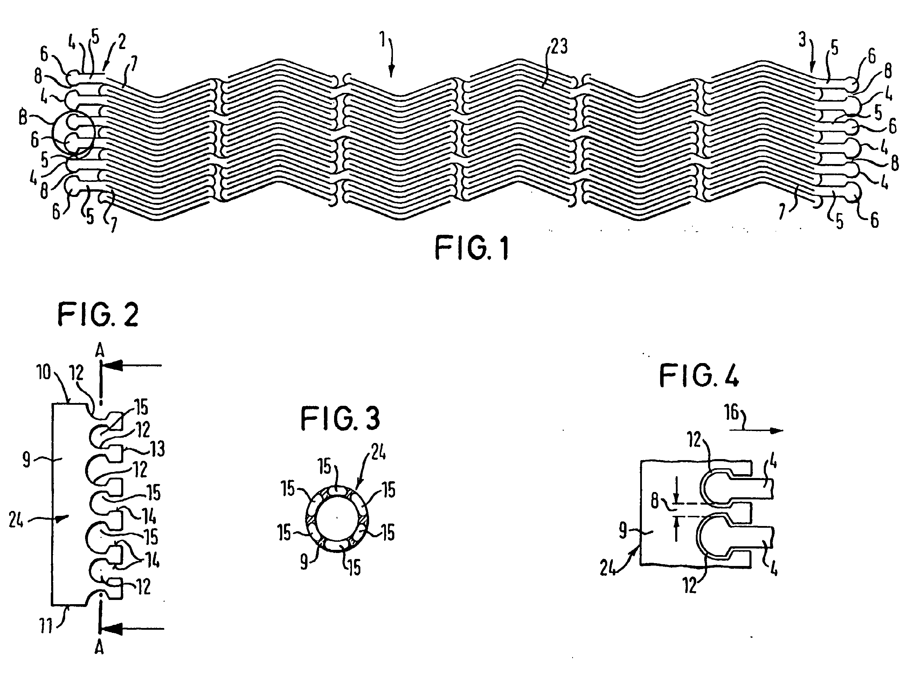

Fig. 1 zeigt eine Abwicklung bzw. ein Schnittmuster eines erfindungsgemäß ausgebildeten Stents 1. Der Stent kann beispielsweise unmittelbar aus einem röhrenförmigen Körper erzeugt werden, indem das in Fig. 1 dargestellte Schnittmuster beispielsweise mit einem Laserstrahl in den röhrenförmigen Körper geschnitten wird. Grundsätzlich ist es auch möglich, daß der Stent im flachen Zustand, beispielsweise durch Laserschneiden, hergestellt wird und anschließend zu einem röhrenförmigen Körper gerollt wird. Weiterhin ist es auch möglich, den Stent beispielsweise aus einem Drahtgeflecht oder -gewirk herzustellen.

Der Stent 1 umfaßt einen Grundkörper 23, an dessen Enden 2, 3 in Umfangsrichtung verteilt mehrere Halteelemente 4 ausgebildet sind. Die Halteelemente 4 umfassen jeweils einen sich in Längsrichtung des Stents 1 erstreckenden, schmalen geraden Verbindungsabschnitt 5 sowie einen sich daran anschließenden runden Halteabschnitt 6. Die Verbindungsabschnitte 5 sind jeweils als Fortführungen von Stegen 7 ausgebildet, die eine gitterförmige Skelettstruktur des Grundkörpers 23 des Stents 1 erzeugen. Gemäß Fig. 1 sind jeweils zwei Stege 7 an ihren Enden so zusammengefaßt, daß ihre Verlängerung jeweils einen Verbindungsabschnitt 5 bildet.

Die Halteabschnitte 6 weisen in Umfangsrichtung eine größere Breite auf als die Verbindungsabschnitte 5, so daß die Halteelemente 4 eine pilzförmige Außenkontur besitzen.

Zwischen benachbart angeordneten Halteabschnitten 6 ist jeweils ein Freiraum 8 ausgebildet, der auch bei vollständig komprimiertem Stent 1 vorhanden ist, wenn in Umfangsrichtung benachbart angeordnete Stege 7 aneinander anliegen, wie es in Fig. 1 gezeigt ist. Um einen möglichst großen Freiraum 8 zu erhalten, besitzen die Halteabschnitte 6 alternierend in Umfangsrichtung jeweils unterschiedliche Größen. Grundsätzlich ist es auch möglich, daß beispielsweise jedes zweite Halteelement 4 entfällt und dadurch die Halteabschnitte 6 der verbleibenden Halteelemente 4 noch größer als in Fig. 1 dargestellt ausgebildet werden können, wobei gleichzeitig der Freiraum 8 zwischen benachbarten Halteabschnitten 6 erhalten bleibt.

Fig. 2 zeigt die Abwicklung eines als ringförmiger Körper 9 ausgebildeten Positionierelements 24. Zur Bildung des ringförmigen Körpers 9 kann die in Fig. 2 dargestellte Abwicklung beispielsweise zu einem Ring gebogen und die beiden Enden 10, 11 des ringförmigen Körpers 9 miteinander verbunden, beispielsweise verschweißt oder verlötet werden. Es ist jedoch auch möglich, den ringförmigen Körper 9 unmittelbar aus einem Rohr herzustellen, beispielsweise ein entsprechendes ringförmiges Teil abzuschneiden.

In der Fläche des ringförmigen Körpers 9 sind eine Vielzahl von Ausnehmungen 12 ausgebildet, die jeweils Aufnahmeabschnitte für die Halteelemente 4 des Stents 1 nach Fig. 1 bilden. Bei der erfindungsgemäßen Ausführungsform gemäß Fig. 2 sind die Ausnehmungen 12 als Durchbrechungen ausgebildet, die sich jeweils bis zum dem stirnseitigen Ende 13 erstrecken und dort in die Umgebung münden. Die Ausnehmungen 12 besitzen dabei an ihren stirnseitigen Austrittsenden 14 jeweils in Umfangsrichtung eine geringere Breite als in ihren von dem stirnseitigen Ende 13 abgewandten Bereichen 15.

Gemäß dem Ausführungsbeispiel nach Fig. 2 besitzen die Ausnehmungen 12 eine im wesentlichen pilzförmige Außenkontur, die komplementär zu der Außenkontur der Halteelemente 4 nach Fig. 1 ausgebildet ist.

Fig. 3 zeigt einen Schnitt entlang der Linie A-A durch den ringförmigen Körper 9 aus Fig. 2, aus der die unterschiedlichen Breiten der Bereiche 15 der Ausnehmungen 12 in Umfangsrichtung erkennbar ist.

In Fig. 4 ist eine Detailansicht eines Bereichs B aus Fig. 1 dargestellt. Dabei ist zusätzlich zu den vergrößert dargestellten Halteelementen 4 ein Teil des ringförmigen Körpers 9 abgebildet.

Die Halteelemente 4 sind so in die Ausnehmungen 12 des ringförmigen Körpers 9 eingesetzt, daß eine Entkopplung der Halteelemente 4 in die durch einen Pfeil 16 dargestellte Längsrichtung des Stents 1 nicht möglich ist. Eine Entnahme der Halteelemente 4 und damit eine Entkopplung von dem ringförmigen Körper 9 ist nur durch Verschieben der Halteelemente 4 in einer Richtung senkrecht zur Bildebene möglich. Durch die zwischen den Halteabschnitten 6 auch im komprimierten Zustand des Stents 1 vorhandenen Freiräume ist gewährleistet, daß auch die Halteelemente 4 und das Positionierelement 24 im komprimierten Zustand des Stents 1 verkoppelt werden können, wie es in Fig. 4 dargestellt ist.

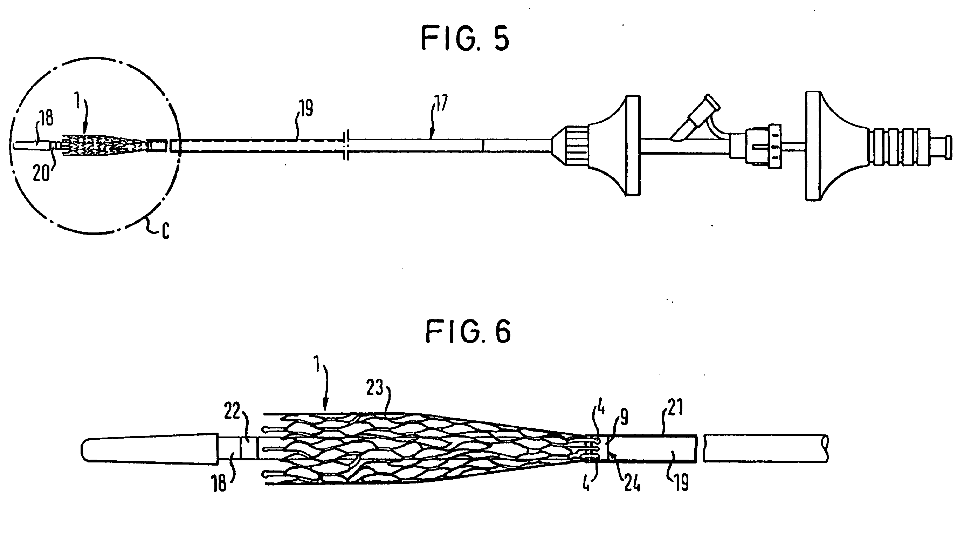

Fig. 5 zeigt einen Einführkatheter 17, der einen Mandrin 18 sowie eine auf den Mandrin 18 aufgeschobene Außenhülle 19 umfaßt. Der Einführkatheter 17 ist dabei im wesentlichen als handelsüblicher Einführkatheter ausgebildet.

Im Bereich des distalen Endes 20 des Mandrins ist auf diesem ein erfindungsgemäßer Stent 1 aufgesetzt, der aufgrund des Zurückziehens der Außenhülle 19 bereits zum Großteil freigesetzt und in seinen expandierten Zustand übergegangen ist. Dies ist in der Detailansicht C gemäß Fig. 6 deutlicher zu erkennen.

Auf dem Mandrin 18 ist der erfindungsgemäß ausgebildete ringförmige Körper 9 unverschiebbar angeordnet, wobei zum Ausgleich der radialen Dicke des ringförmigen Körpers 9 sich an diesen in proximaler Richtung ein Füllmaterial 21 anschließt. Auf diese Weise ist gewährleistet, daß die durch die Außenseiten des ringförmigen Körpers 9 und des Füllmaterials 21 gebildete Oberfläche keine Stufen besitzt, so daß die Außenhülle 19 ohne Probleme auf dem Mandrin 18 verschoben werden kann.

Die am proximalen Ende des Stents 1 angeordneten Halteelemente 4 sind, wie in Fig. 4 gezeigt, in den Ausnehmungen 12 des ringförmigen Körpers 9 eingesetzt, so daß eine Entkopplung der Halteelemente 4 von dem ringförmigen Körper 9 in Längsrichtung des Einführkatheters 17 nicht möglich ist.

Eine radiale Entkopplung der Halteelemente 4 wird durch die Außenhülle 19 verhindert, durch die die Halteelemente 4 in den Ausnehmungen 12 des ringförmigen Körpers 9 gehalten werden. Solange die Außenhülle 19 zumindest noch das proximale Ende des Stents 1 mit den Halteelementen 4 umschließt, wird ein unbeabsichtigtes Absetzen des Stents 1 zuverlässig verhindert. Weiterhin kann in diesem Fall durch Zurückziehen des Mandrins 18 der Stent 1 nochmals vollständig in die Außenhülle 19 eingezogen werden, so daß er repositioniert werden kann.

Da der ringförmige Körper 9 aus einem röntgendichten Material, beispielsweise aus Platin, hergestellt ist, kann bei der Positionierung des Stents dessen proximales Ende gut am Röntgenbildschirm verfolgt werden. Zusätzlich ist an dem Mandrin 18 ein weiterer Ring 22 aus röntgendichten Material vorgesehen, der in Höhe des distalen Endes des Stents 1 angeordnet ist. Durch Beobachtung dieses weiteren Rings 22 kann auch die Position des distalen Endes des Stents 1 beim Positionieren am Röntgenschirm deutlich erkannt werden.

### Bezugszeichenliste

- 1: Stent
- 2: Ende des Stents
- 3: Ende des Stents
- 4: Halteelemente
- 5: Verbindungsabschnitt
- 6: Halteabschnitt
- 7: Stege
- 8: Freiraum
- 9: ringförmiger Körper
- 10: Ende des ringförmigen Körpers
- 11: Ende des ringförmigen Körpers
- 12: Ausnehmungen
- 13: stimseitiges Ende des ringförmigen Körpers
- 14: Austrittsenden
- 15: verbreitete Bereiche
- 16: Pfeil
- 17: Einführkatheter
- 18: Mandrin
- 19: Außenhülle
- 20: distales Ende des Mandrins
- 21: Füllmaterial
- 22: Ring
- 23: Grundkörper
- 24: Positionierelement

## Patentansprüche

1. Einführkatheter für einen von einem komprimierten in einen expandierten Zustand überführbaren, zumindest an einem Ende mit Halteelementen (4) versehenen Stent (1) mit einer Außenhülle (19), in die der Stent (1) einschiebbar ist, und mit einem Positionierelement (24) mit einem ringförmigen Körper (9), in dem in Umfangsrichtung verteilt mehrere, Aufnahmeabschnitte für die Halteelemente (4) bildende Ausnehmungen (12) ausgebildet sind, die sich jeweils bis zu einem stirnseitigen Ende (13) des ringförmigen Körpers (9) erstrecken und dort in die Umgebung münden, wobei jeweils der zur Mitte des ringförmigen Körpers (9) hin gelegene Bereich (15) der Ausnehmungen (12) in Umfangsrichtung eine größere Breite aufweist als der zum stirnseitigen Ende (13) hin gelegene Bereich (14),
**dadurch gekennzeichnet ,**
**dass** ein innen liegender Mandrin (18) vorgesehen ist, auf den der Stent (1) im komprimierten Zustand aufsetzbar ist,
**dass** der Mandrin (18) zusammen mit dem Stent (1) in die Außenhülle (19) einschiebbar ist,
**dass** das Positionierelement (24) auf dem Mandrin (18) unverschiebbar angeordnet ist und
**dass** die Ausnehmungen (12) als Durchbrechungen ausgebildet sind.

2. Einführkatheter nach Anspruch 1,
**dadurch gekennzeichnet,**
**dass** die radiale Dicke des ringförmigen Körpers (9) im wesentlichen gleich der radialen Dicke der Halteelemente (4) und/oder Wanddicke des Stents (1) ist.

3. Einführkatheter nach Anspruch 1 oder 2,
**dadurch gekennzeichnet ,**
**dass** der Durchmesser des ringförmigen Körpers (9) des Positionierelements (24) im wesentlichen gleich dem Durchmesser des Stents (1) im komprimierten Zustand ist.

4. Einführkatheter nach einem der vorhergehenden Ansprüche,
**dadurch gekennzeichnet ,**
**dass** die Entfernung zwischen dem distalen Ende des Mandrins (18) und dem Positionierelement (24) größer ist als die Länge des komprimierten Stents (1).

5. Einführkatheter nach einem der vorhergehenden Ansprüche,
**dadurch gekennzeichnet ,**
**dass** alle Ausnehmungen (12) an demselben stirnseitigen Ende (13) des ringförmigen Körpers (9) in die Umgebung münden.

6. Einführkatheter nach einem der vorhergehenden Ansprüche,
**dadurch gekennzeichnet ,**
**dass** die Ausnehmungen (12) im wesentlichen komplementär zu der Form der Halteelemente (4) des Stents (1) ausgebildet sind.

7. Einführkatheter nach einem der vorhergehenden Ansprüche,
**dadurch gekennzeichnet ,**
**dass** die Durchbrechungen (12) eine abgerundete Kontur besitzen.

8. Einführkatheter nach einem der vorhergehenden Ansprüche,
**dadurch gekennzeichnet ,**
**dass** die Kontur der Durchbrechungen (12) pilzförmig ausgebildet ist.

9. Einführkatheter nach einem der vorhergehenden Ansprüche,
**dadurch gekennzeichnet ,**
**dass** zumindest ein Teil der Durchbrechungen (12) unterschiedlich groß ausgebildet ist.

10. Einführkatheter nach einem der Ansprüche 1 bis 8,
**dadurch gekennzeichnet ,**
**dass** alle Durchbrechungen (12) im wesentlichen gleich groß sind.

11. Einführkatheter nach einem der vorhergehenden Ansprüche,
**dadurch gekennzeichnet ,**
**dass** der ringförmige Körper (9) aus einem im wesentlichen röntgendichten Material besteht.

12. Einführkatheter nach einem der vorhergehenden Ansprüche,
**dadurch gekennzeichnet ,**
**dass** die Anzahl der Durchbrechungen (12) gleich der Anzahl der an dem Stent (1) vorgesehenen Halteelemente (4) ist.

13. Einführkatheter nach einem der vorhergehenden Ansprüche,
**dadurch gekennzeichnet ,**
**dass** der Abstand benachbarter Durchbrechungen (12) in Umfangsrichtung gleich dem Abstand benachbarter Halteelemente (4) des Stents (1) in Umfangsrichtung ist.

14. Einführkatheter nach einem der vorhergehenden Ansprüche,
**dadurch gekennzeichnet ,**
**dass** zumindest ein Teil der Durchbrechungen (12), insbesondere alle Durchbrechungen voneinander getrennt ausgebildet sind.

## Claims

1. A delivery catheter for a stent (1) which can be transformed from a compressed state into an expanded state, which is provided with holding elements (4) at at least one end and which has an outer envelope (19) into which the stent (1) can be inserted and which has a positioning element (24) having a ring-shaped body (9) in which a plurality of recesses (12) forming receiving sections for the holding elements (4) are formed distributed in the peripheral direction which extend in each case up to an end face end (13) of the ring-shaped body (9) and open there into the environment, with in each case the region (15) of the recesses (12) disposed towards the centre of the ring-shaped body (9) having a larger width in the peripheral direction than the region (14) disposed towards the end face end (13),
**characterised in that**
an inwardly disposed mandrel (18) is provided onto which the stent (1) can be placed in the compressed state;
**in that** the mandrel (18) can be inserted into the outer envelope (19) together with the stent (1);
**in that** the positioning element (24) is non-displaceably arranged on the mandrel (18); and
**in that** the recesses (12) are made as openings.

2. A delivery catheter in accordance with claim 1, **characterised in that** the radial thickness of the ring-shaped body (9) is substantially equal to the radial thickness of the holding elements (4) and/or the wall thickness of the stent (1).

3. A delivery catheter in accordance with claim 1 or claim 2, **characterised in that** the diameter of the ring-shaped body (9) of the positioning element (24) is substantially equal to the diameter of the stent (1) in the compressed state.

4. A delivery catheter in accordance with any one of the preceding claims, **characterised in that** the distance between the distal end of the mandrel (18) and the positioning element (24) is larger than the length of the compressed stent (1).

5. A delivery catheter in accordance with any one of the preceding claims, **characterised in that** all the recesses (12) open into the environment at the same end face end (13) of the ring-shaped body (9).

6. A delivery catheter in accordance with any one of the preceding claims, **characterised in that** the recesses (12) are made substantially complementary to the shape of the holding elements (4) of the stent (1).

7. A delivery catheter in accordance with any one of the preceding claims, **characterised in that** the openings (12) have a rounded contour.

8. A delivery catheter in accordance with any one of the preceding claims, **characterised in that** the contour of the openings (12) is made in mushroom shape.

9. A delivery catheter in accordance with any one of the preceding claims, **characterised in that** at least some of the openings (12) are made in different sizes.

10. A delivery catheter in accordance with any one of the claims 1 to 8, **characterised in that** all the openings (12) are substantially of the same size.

11. A delivery catheter in accordance with any one of the preceding claims, **characterised in that** the ring-shaped body (9) consists of a material substantially oblique to X-rays.

12. A delivery catheter in accordance with any one of the preceding claims, **characterised in that** the number of the openings (12) is equal to the number of the holding elements (4) provided at the stent (1).

13. A delivery catheter in accordance with any one of the preceding claims, **characterised in that** the spacing of adjacent openings (12) in the peripheral direction is equal to the spacing of adjacent holding elements (4) of the stent (1) in the peripheral direction.

14. A delivery catheter in accordance with any one of the preceding claims, **characterised in that** at least some of the openings (12), in particular all of the openings, are made separately from one another.

## Revendications

1. Cathéter d'insertion pour un stent susceptible d'être transféré d'un état comprimé vers un état expansé et pourvu, à au moins une extrémité, d'éléments de maintien (4) et présentant une enveloppe extérieure (19) dans laquelle on peut insérer le stent (1), et comportant un élément de positionnement (24) pourvu d'un corps annulaire (9) dans lequel sont ménagés, en répartition en direction périphérique, plusieurs évidements (12) formant des tronçons de logement pour les éléments de maintien (4), qui s'étendent chacun jusqu'à une extrémité (13) côté frontal du corps annulaire (9) et qui débouchent ici dans l'environnement, la zone respective (15) des évidements (12) située vers le milieu du corps annulaire (9) présentant en direction périphérique une largeur supérieure à celle de la zone (14) située vers l'extrémité (13) côté frontal,
**caractérisé en ce que**
il est prévu un mandrin (18) situé à l'intérieur, sur lequel le stent (1) peut être rapporté dans l'état comprimé,
le mandrin (18) est susceptible d'être introduit conjointement avec le stent (1) dans l'enveloppe extérieure (19),
l'élément de positionnement (24) est agencé de façon immobile sur le mandrin (18), et
les évidements (12) sont réalisés sous forme de traversées.

2. Cathéter d'insertion selon la revendication 1,
**caractérisé en ce que** l'épaisseur radiale du corps annulaire (9) est sensiblement égale à l'épaisseur radiale des éléments de maintien (4) et/ou à l'épaisseur de paroi du stent (1).

3. Cathéter d'insertion selon la revendication 1 ou 2,
**caractérisé en ce que**
le diamètre du corps annulaire (9) de l'élément de positionnement (24) est sensiblement égal au diamètre du stent (1) dans l'état comprimé.

4. Cathéter d'insertion selon l'une des revendications précédentes,
**caractérisé en ce que**
la distance entre l'extrémité distale du mandrin (18) et l'élément de positionnement (24) est supérieure à la longueur du stent comprimé (1).

5. Cathéter d'insertion selon l'une des revendications précédentes,
**caractérisé en ce que**
tous les évidements (12) débouchent dans l'environnement à la même extrémité (13) côté frontal du corps annulaire (9).

6. Cathéter d'insertion selon l'une des revendications précédentes,
**caractérisé en ce que**
les évidements (12) sont réalisés de façon sensiblement complémentaire à la forme des éléments de maintien (4) du stent (1).

7. Cathéter d'insertion selon l'une des revendications précédentes,
**caractérisé en ce que**
les traversées (12) possèdent un contour arrondi.

8. Cathéter d'insertion selon l'une des revendications précédentes,
**caractérisé en ce que**
le contour des traversées (12) est réalisé en forme de champignon.

9. Cathéter d'insertion selon l'une des revendications précédentes,
**caractérisé en ce que**
une partie au moins des traversées (12) sont réalisées avec différentes tailles.

10. Cathéter d'insertion selon l'une des revendications 1 à 8,
**caractérisé en ce que**
toutes les traversées (12) présentent sensiblement la même taille.

11. Cathéter d'insertion selon l'une des revendications précédentes,
**caractérisé en ce que**
le corps annulaire (9) est constitué d'un matériau sensiblement imperméable aux rayons X.

12. Cathéter d'insertion selon l'une des revendications précédentes,
**caractérisé en ce que**
le nombre des traversées (12) est égal au nombre des éléments de maintien (4) prévus sur le stent (1).

13. Cathéter d'insertion selon l'une des revendications précédentes,
**caractérisé en ce que**
la distance de traversées voisines (12) en direction périphérique est égale à la distance d'éléments de maintien voisins (4) du stent (1) en direction périphérique.

14. Cathéter d'insertion selon l'une des revendications précédentes,
**caractérisé en ce que**
une partie au moins des traversées (12), en particulier toutes les traversées, sont réalisées de façon séparée les unes des autres.
